# EUROPÄISCHE PATENTSCHRIFT

(11) **EP 0 756 593 B1**
(45) Veröffentlichungstag und Bekanntmachung des Hinweises auf die Patenterteilung: **20.09.2000**
(21) Anmeldenummer: 95918571.1
(22) Anmeldetag: 19.04.1995
(51) Int. Cl.: C07D 241/04, C07D 498/04

(54) **VERFAHREN ZUR HERSTELLUNG VON 2-PIPERAZINCARBONSÄUREDERIVATEN**
PROCESS FOR PREPARING 2-PIPERAZINE CARBOXYLIC ACID DERIVATIVES
PROCEDE POUR PREPARER DES DERIVES D'ACIDE 2-PIPERAZINE CARBOXYLIQUE

(30) Priorität: 20.04.1994 CH 120594
(43) Veröffentlichungstag der Anmeldung: 05.02.1997
(73) Patentinhaber: LONZA AG, CH-3945 Gampel/Wallis (CH)
(72) Erfinder: BRIEDEN, Walter, CH-3902 Glis (CH); RODUIT, Jean-Paul, CH-3979 Grône (CH)
(74) Vertreter: Weinhold, Peter, Dr.rer.nat. Dipl.-Chem.
(86) Internationale Anmeldenummer: EP9501475
(87) Internationale Veröffentlichungsnummer: WO9529169

(56) Entgegenhaltungen:
- EP-A- 0 068 544
- EP-A- 0 285 219
- INDIAN J. CHEM., Bd. 13, Nr. 5, 1975 Seiten 468-472, S. SHARMA ET AL. 'Studies in Potential Filaricides: Part VII - Synthesis of 4,8-Dialkyl-1,4,8-triazabicyclo(4.4.0)deca n-2-one, 3,7-Dialkyl-1,3,7-triazabicyclo(4.3.0)nona n-2-one & Related Oxygen Analogs'
- INDIAN J. CHEM., SECT. B, Bd. 16B, Nr. 11, 1978 Seiten 1015-1018, Y. KHANDELWAL, P. C. JAIN 'Agents Acting on CNS: Part XXXV - Synthesis of Imidazo(1,5-a)pyrazines & Pyrazino(1,2-a)pyrazines'

## Beschreibung

Die Erfindung betrifft ein neues Verfahren zur Herstellung von 2-Piperazincarbonsäureamiden der allgemeinen Formel 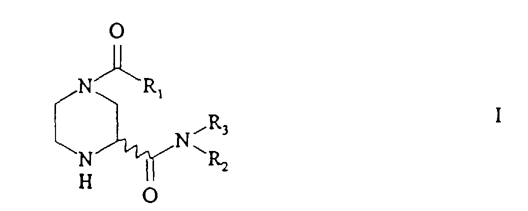 worin R₁ die Bedeutung hat von a) gegebenenfalls substituiertes Alkyl oder b) von -OR₄, worin R₄ gegebenenfalls substituiertes Alkyl, Alkenyl oder Aryl bedeutet oder c) von -NR₅R₆, worin R₅ Wasserstoff oder Alkyl und R₆ Alkyl bedeutet und
R₂ und R₃ gleich oder verschieden sind und die Bedeutung haben von Wasserstoff, gegebenenfalls substituiertes Alkyl, Alkenyl oder Aryl oder für den Rest einer Aminosäure oder eines Aminosäureesters stehen.

Mit dem erfindungsgemässen Verfahren sind sowohl die (R)- oder (S)-Enantiomeren der 2-Piperazincarbonsäureamide als auch die Mischungen der Enantiomeren z. B. die Racemate zugänglich. Die 2-Piperazincarbonsäureamide sind u.a. wichtige Zwischenprodukte zur Herstellung von oral aktiven HIV-1 Protease Inhibitoren. (EP-A 541 168)

In der bisher bekannten Synthese gemäss EP-A 541 168 erfolgt gemäss Beispiel 15 die Herstellung des N-(t-Butyl)-4-(t-butoxycarbonyl)-2(S)-piperazincarboxamids der Formel 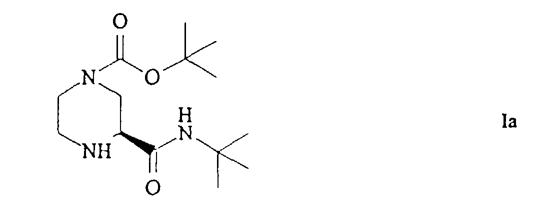 ausgehend von der 2-(S)-Piperazincarbonsäure durch Überführung in die 1-Z-geschützte und 4-BOC-geschützte 2-(S)-Piperazincarbonsäure, durch Bildung des tert. Butylamids und schliesslich durch katalytische Hydrierung zum Entfernen der Z-Schutzgruppe.

Diese Synthese ist sehr aufwendig und eignet sich deshalb lediglich für den Labormassstab. Aus Tetrahedron Letters 1994, 35, 676 ist ausserdem bekannt die Verbindung der Formel Ia in einer "overall"-Ausbeute von 26% ausgehend von 2-Pyrazincarbonsäure, durch Weiterumsetzung in das t-Butylamid, Hydrierung zum Piperazincarbonsäureamid, Racemattrennung mit Camphersulfonsäure und schliesslich durch Einführung der BOC-Schutzgruppe herzustellen. Auch diese Synthese ist für eine Übertragung in den technischen Massstab nicht geeignet.

Es bestand daher die Aufgabe eine Synthese zu entwickeln, die erlaubt die Piperazincarbonsäureamide einfach, mit guten Ausbeuten und im technischen Massstab, herzustellen.

Die Aufgabe konnte gelöst werden mit dem erfindungsgemässen Verfahren gemäss Anspruch 1.

Die erste Stufe dieses Verfahrens beinhaltet die Umwandlung einer 2-Piperazincarbonsäure der Formel 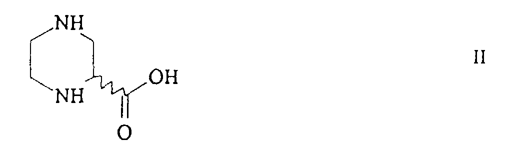 oder von einem Salz davon in ein N-Acylderivat der allgemeinen Formel 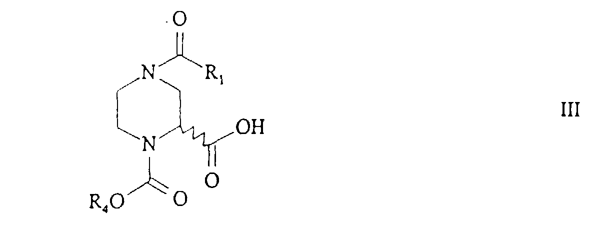 worin R₁ und R₄ die genannte Bedeutung haben.

Alkyl steht zweckmässig für eine geradkettige oder verzweigte, gegebenenfalls substituierte Alkylgruppe mit 1 bis 6 C-Atomen.
Für Alkyl sind beispielhaft Methyl, Ethyl, n- oder i-Propyl, n-, i- oder t-Butyl, Pentyl und seine Isomeren oder Hexyl und seine Isomeren genannt.
Geeignete Alkenylgruppen sind Vinyl, 1- oder 2-Propenyl (Allyl), 1-, 2- oder 3-Butenyl, Pentenyl und seine Isomeren oder Hexenyl und seine Isomeren.
Aryl ist zweckmässig eine gegebenenfalls substituierte Phenyl- oder Naphthylgruppe.
Als Substituenten der Alkyl- oder Alkenylgruppe sind vorallem Phenyl oder Halogene wie Chlor oder Brom zu nennen. Bevorzugter Vertreter einer substituierten Alkylgruppe ist Benzyl.
Als Arylsubstituenten können Halogene wie Chlor oder Brom oder die genannten Alkylgruppen auftreten.
Bevorzugt hat R₁ die Bedeutung von -OR₄, worin R₄ die genannte Bedeutung hat. R₄ hat bevorzugt die Bedeutung von Benzyl, von t-Butyl oder von Allyl.

Die N-Acylierung zu den N-Acylderivaten der allgemeinen Formel III kann mit für die Blockierung von Aminogruppen bekannten Acylierungsreagentien und bei bekannten Bedingungen erfolgen (s. z. B. Houben-Weyl, Methoden der org. Chemie 4. Auflage, Bd. 15/1, Synthese von Peptiden S. 46ff).

Für die Herstellung der genannten bevorzugten N-Acylderivate werden als Acylierungsreagentien z. B. der Chlorameisensäurebenzylester für R₄ = Benzyl das Di-t-Butyldicarbonat für R₄ = t-Butyl und z. B. der Chlorameisensäureallylester für R₄ = Allyl eingesetzt.

In den Fällen wo R₁ nicht -OR₄ bedeutet, kann vor dem Acylierungsschritt, entsprechend der weiteren Bedeutungen von R₁ und auf bekannte Weise, am N-4-Stickstoff der 2-Piperazincarbonsäure zunächst eine Alkanoylfunktion wie z. B. Acetyl oder eine Carbonsäureamidfunktion eingeführt werden.

Die N-Acylierung verläuft praktisch quantitativ. Das resultierende N-Acylderivat der allgemeinen Formel III wird zweckmässig, ohne es zu isolieren, in Gegenwart von einem Halogenierungsmittel zu einem Piperazincarbonsäureanhydrid der allgemeinen Formel 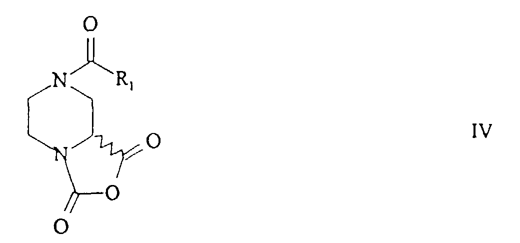 worin R₁ die genannte Bedeutung hat, cyclisiert.

Als Halogenierungsmittel werden zweckmässig Thionylchlorid, Phosphorhalogenide wie Phosphor (V)-chlorid, Phosgen oder Phosgenderivate wie Di- und Triphosgen eingesetzt. Vorzugsweise wird Thionylchlorid verwendet.

Vorteilhaft wird die Cyclisierung in Gegenwart von katalytischen Mengen von 0,1 mol% bis 30 mol% eines N,N-disubstituierten Formamids bezogen auf das Holgenierungsrmittel durchgeführt.
Geeignete N,N-disubstituierte Formamide sind beispielsweise das N,N-Dibenzylformamid, das N,N-Diisopropylformamid, das N,N-Dimethylformamid oder das N,N-Diethylformamid. Bevorzugt wird N,N-Dimethylformamid verwendet.

Die N,N-disubstituierten Formamide im Überschuss eingesetzt, können aber auch zusätzlich die Funktion des Lösungsmittels einnehmen. Es ist zweifellos möglich auch inerte Lösungsmittel wie z. B. Tetrahydrofuran oder Acetonitril einzusetzen.

Zum Auffangen des entstehenden Halogenwasserstoffs wird zweckmässig ein tertiäres Amin wie z. B. Pyridin oder Triethylamin zugesetzt.
Die Reaktionstemperatur liegt zweckmässig im Bereich zwischen 0° und 100°C.

Alternativ ist es möglich entsprechend J. Org. Chem. 1985, 50, 2200 ff. das N-Acylderivat der allgemeinen Formel III mit einem Halogensilan in einen Silylester zu überführen und diesen Silylester mit einem Halogenierungsmittel zum Anhydrid der allgemeinen Formel IV zu cyclisieren.
Die erstgenannte Variante wird allerdings bevorzugt.

Das resultierende Piperazincarbonsäureanhydrid der allgemeinen Formel IV kann nach fachmännischer Aufarbeitung, zweckmässig durch Extraktion mit einem geeigneten Lösungsmittel, aus dem Reaktionsgemisch isoliert werden. Es ist aber auch möglich ohne Isolierung dieser Zwischenstufe direkt mit der Folgestufe fortzufahren.

Das Piperazincarbonsäureanhydrid der genannten Formel IV ist nicht literaturbekannt und als wichtiges Zwischenprodukt der erfindungsgemässen Synthese ebenfalls Bestandteil der Erfindung.

Bevorzugte Verbindungen der allgemeinen Formel IV sind die Piperazincarbonsäureanhydride mit R₁ = t-Butyloxy, R₁ = Benzyloxy oder R₁ = Allyloxy, in Form seiner Enantiomeren oder seiner Enantiomerenmischungen z. B. der Racemate. Besonders bevorzugte Verbindungen sind (R)-1,3-Dioxotetrahydrooxazo[3,4-a]piperazin-7-carbonsäure-t-butylester, (S)-1,3-Dioxotetrahydrooxazo[3,4-a]piperazin-7-carbonsäure-t-butylester, (R)-1,3-Dioxotetrahydrooxazo[3,4-a]-piperazin-7-carbonsäurebenzylester, (S)-1,3-Dioxotetrahydrooxazo[3,4-a]piperazin-7-carbonsäurebenzylester, (R)-1,3-Dioxotetrahydrooxazo[3,4-a]piperazin-7-carbonsäureallylester, (S)-1,3-Dioxotetrahydrooxazo[3,4-a]piperazin-7-carbonsäureallylester.

In der letzten Stufe des erfindungsgemässen Verfahrens wird das Piperazincarbonsäureanhydrid der allgemeinen Formel IV mit einem Amin der allgemeinen Formel 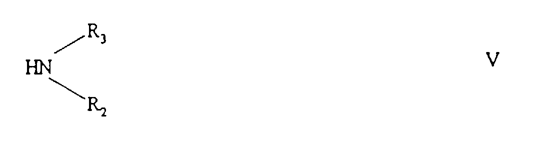 worin R₂ und R₃ die genannte Bedeutung haben zum Endprodukt der allgemeinen Formel 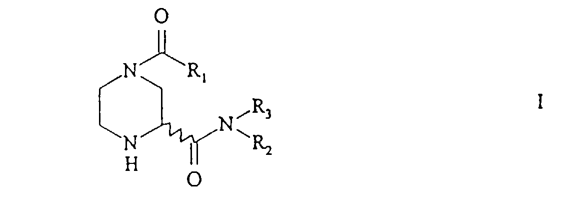 worin R₁, R₂ und R₃ die genannte Bedeutung haben, umgesetzt.

R₂ und R₃ als Alkyl-, Alkenyl- oder Arylgruppe haben zweckmässig die vorstehend genannten näheren Bedeutungen.
Bevorzugt steht R₂ für gegebenenfalls substituiertes (C₁-C₆)-Alkyl oder (C₂-C₆)-Alkenyl und R₃ für Wasserstoff.
Insbesondere steht R₂ für t-Butyl, Allyl, Benzyl oder für 1-Phenylethyl, 1-Phenylethyl kann dabei racemisch oder in Form seines 1-(S)- oder 1-(R)Enantiomeren auftreten.

R₂ oder R₃ in der Bedeutung als Rest einer Aminosäure oder eines Aminosäureesters umfassen im Prinzip alle Reste von Aminosäuren, die die Aminogruppe in z. B. α-Stellung, β-Stellung oder endständig zur Carboxylgruppe bzw. zur Estergruppe enthalten. Für eine Übersicht sei auf Kapitel 11 von Houben Weyl, Methoden der org. Chemie 4. Auflage Bd. 15/1, Synthese von Peptiden hingewiesen.
Bevorzugst steht R₂ für den Rest einer der 20 essentiellen L-α-Aminosäuren d. h. für Alanin, Valin, Leucin, Isoleucin, Prolin, Tryptophan, Phenylalanin, Methionin, Glycin, Serin, Tyrosin, Threonin, Cystein, Asparagin, Glutamin, Aspartat, Glutamat, Lysin, Arginin oder Histidin oder deren (C₁-C₄)-Alkylester und R₃ für Wasserstoff .

Die Umsetzung in der letzten Stufe verläuft vorteilhaft in Gegenwart eines inerten Lösungsmittels wie z B. Dichlormethan oder wässrigen alkalischen Lösungen bei einer Temperatur im Bereich von -20°C bis Rückflusstemperatur des Lösungsmittels.
Nach relativ kurzer Umsetzungszeit kann das gewünschte 2-Piperazincarbonsäureamid der allgemeinen Formel I in einer "overall" Ausbeute, bezogen auf die eingesetzte 2-Piperzincarbonsäure, von über 60% erhalten werden.

### Beispiel 1

### a1) Herstellung von (S)-Piperazin-1,2,4-tricarbonsäure-1,4-di-(t-butylester)

Zu einer Aufschlämmung von 19,30 g (91,2 mmol) 2-(S)-Piperazincarbonsäure-dihydrochlorid (96%) in 100 ml Methanol wurden 38,0 ml (273 mmol) Triethylamin zugegeben und eine Lösung von 50,00 g (229 mmol) Di-tert-Butyldicarbonat in 100 ml Methanol innerhalb von 20 min zugetropft. Nach dem Rühren bei 50°C über Nacht wurde bis zur Trockene eingedampft und der Rückstand mit 250 ml Wasser versetzt. Mit 1N Salzsäure wurde die Mischung auf pH = 2 eingestellt und viermal mit 150 ml Essigsäureethylester extrahiert. Die vereinigten organischen Extrakte wurden über Magnesiumsulfat getrocknet. Das Filtrat wurde auf ca. 100 ml eingeengt und mit 100 ml n-Hexan versetzt. Bei 0°C wurde das Produkt abfiltriert, mit wenig n-Hexan gewaschen und getrocknet. 27,90 g (92%) der Titelverbindung wurde als weisser, kristalliner Feststoff isoliert.
[α]_{D}²⁰ = -17 6° (C = 1; Methanol)
Fp. = 143,0 - 144,5°C
¹H-NMR (CDCl₃, 400 MHz): δ 10,70 (s, br, 1H),
4,78 - 4,50 (m, 2H);
4,10 - 3,72 (m, 2H);
3,29 - 3,06 (m, 2 H);
2,94 - 2,78 (m, 1 H),
1,52 - 1,42 (m, 18 H).

### a2) Herstellung von (R)-Piperazin-1,2,4-tricarbonsäure1,4-di-(t-butylester)

Ausgehend von 2-(R)-Piperazincarbonsäure-dihydrochlorid wurde in einer Ausbeute von 92% die Titelverbindung hergestellt.
[α]_{D}²⁰ = +18,5° (C = 1; Methanol)

### a3) Herstellung von Piperazin-1,2,4-tricarbonsäure-1,4-diallylester

Zu 5,00 g (24,6 mmol) 2(R,S)-Piperazincarbonsäure-dihydrochlorid in 60 ml Aceton und 30 ml Wasser wurde 10,20 g (73,80 mmol) Kaliumcarbonat zugegeben und auf 63 °C erhitzt. bei dieser Temperatur wurde 9,50 g (78,8 mmol) Chlorameisensäureallylester zugetropft und 2,5 h bei 63 °C gehalten. Zur Aufarbeitung wurde nach dem Erkalten mit 50 ml Wasser versetzt und mit 5 N Salzsäure auf pH = 2 gestellt und anschließend bis zur Trockne einrotiert. Dreimalige Extraktion mit je 80 ml Essigsäureethylester ergab 5,10 g (70 %) der reinen Titelverbindung als hellgelbes Öl.
¹H-NMR (DMSO-d₆, 400 MHz): δ 13,20 - 12,90 (m, 1H),
6,00 - 5,83 (m, 2H),
5,38 - 5,15 (m, 4H),
4,64 - 4,48 (m, 5 H),
4,49 - 4,38 ("t", 1H),
3,96 - 3,88 (m, 1H),
3,88 - 3,78 (m, 1H),
3,30 - 3,01 (m, 2H),
3,00 - 2,88 (m, 1H).

¹³C-NMR (DMSO-d₆, 100 MHz): δ 171,30 (s),
171,18 (s),
155,29 (s),
154,99 (s),
154,22 (s),
154,18 (s),
133,29 - 132,90 (d),
117,22 - 116,79 (t),
65,63 - 65,30 (t),
53,98 (d),
53,54 (d),
44,06 (t),
43,87 (t),
43,09 (t),
42,46 (t),
40,69 (t),
40,43 (t).

### b1) Herstellung von (S)-1,3-Dioxotetrahydrooxazol[3,4-a]piperazin-7-carbonsäure-t-butylester

Zu einer Suspension von 10,00 g (30,3 mmol) des Produktes aus Beispiel 1a1) und 50 ml Tetrahydrofuran wurden 3,60 g (45,5 mmol) Pyridin, 0,73 g (10 mmol) N,N-Dimethylformamid und 4,80 g (40,3 mmol) Thionylchlorid nacheinander zugegeben. Nach 4stündigem Rühren bei 40°C wurde durch Zugabe von 300 ml Essigsäureethylester und 150 ml Wasser aufgearbeitet. die wässrige Phase wurde noch zweimal mit je 150 ml Essigsäureethylester extrahiert. Die vereinigten organischen Phasen wurden über Magnesiumsulfat getrocknet und eingeengt. Der Rückstand wurde mit 20 ml Diethylether versetzt und bei 0°C gekühlt. Das abfiltrierte Produkt wurde noch zweimal mit je 10 ml Diethylether gewaschen. Nach dem Trocknen wurden 6,30 g (81%) des Titelproduktes als weisser, kristalliner Feststoff isoliert.
[α]_{D}²⁰ = -24,2° (C = 1; Dichlormethan)
Fp. = 116 - 117°C (Zersetzung)
¹H-NMR (CDCl₃, 400 MHz): δ 4,65 - 4,50 (s, br, 1H);
4,23 - 4,19 ("dd", 2 H);
3,98 (dd, J = 3,4, 13,3 Hz, 1H);
3,12 (dt, J = 3,9, 12,8 Hz, 1H);
2,94 - 2,78 (m, 2 H);
1,49 (s, 9 H).

### b2) Herstellung von -1,3-Dioxotetrahydrooxazol[3,4-a]piperazin-7-carbonsäure-t-butylester

Ausgehend von (R)-Piperazin-1,2,4-tricarbonsäure-1,4-di-(t-butylester) wurde in einer Ausbeute von 85% die Titelverbindung hergestellt.

### b3) Herstellung von 1 3-Dioxotetrahydrooxazol[3,4-a]piperazin-7-carbonsäureallylester

Zu 1,80 g (6,03 mmol) Piperazin-1,2,4-tricarbonsäure-1,4-diallylester in 20 ml Dichlormethan wurde 1,07 g (9,00 mmol) Thionylchlorid gegeben und 2,5 h bei 30 °C gerührt. Anschließend wurde das Lösungsmittel im Vakuum entfernt. Das ¹³C-NMR vom Rohprodukt zeigt die Titelverbindung und Piperazin-1,2,4-tricarbonsäure-1,4-di-allylester.

Die charakteristischen Resonanzen von den Carbonylgruppen der Titelverbindung lauten :
¹³C-NMR (DMSO-d₆, 100 MHz): δ 167.10(s),
154,20 (s),
149,95 (s).

### c1) Herstellung von (S)-3-(tert-Butylcarbamoyl)piperazin-1-carbonsäure-t-butylester

Zu einer Lösung von 1,10 g (15,0 mmol) t-Butylamin in 15 ml Dichlormethan wurden 2,56 g (10,0 mmol) des Produktes aus Beispiel 1b1) in 15 ml Dichlormethan getropft. Nach 45 min unter Rückfluss wurde dreimal mit je 10 ml Wasser ausgeschüttelt und die organische Phase über Magnesiumsulfat getrocknet. Nach Entfernen des Lösungsmittels wurde der erhaltene Rückstand mit 20 ml Diethylether versetzt. Nach dem Abziehen von Diethylether wurden 2,34 g (82%) des Titelproduktes als kristalliner Feststoff isoliert.
[α]²⁵₃₆₅ nm = +102,8° (C = 1; Methanol)
Fp. = 105,0 - 105,5°C
¹H-NMR (CDCl₃, 400 MHz): δ 6,75 - 6,45 (s, br, 1H);
4,13 - 4,02 (m, 1H);
3,90 - 3,70 (s, br, 1 H);
3,20 (dd, J = 3,7, 9,3 Hz, 1H);
3,05 - 2,80 (m, 3 H);
2,80 - 2,70 (m, 1 H);
2,05 - 1,90 (s, br, 1 H);
1,47 (s, 9 H);
1,35 (s, 9 H).

### c2) Herstellung von (R)-3-(tert-Butylcarbarmoyl)piperazin-1-carbonsäure-t-butylester

Ausgehend von (R)-1,3-Dioxotetrahydrooxazol[3,4-a]piperazin-7-carbonsäure-t-butylester wurde in einer Ausbeute von 70% die Titelverbindung hergestellt.
[α]²⁰ = -98,4° (C = 1; Methanol)
Fp. = 103,5 - 105,0°C

### Beispiel 2

### Herstellung von 3(S)-[1(S)-Phenylethylcarbamoyl]piperazin-1-carbonsäure-t-butylester und 3(R)-[1(S)-Phenylethylcarbamoyl]piperazin-1-carbonsäure-t-butylester

Zu 5,20 g (42,9 mmol) (s)-1-Phenylethylamin in 50 ml Dichlormethan wurde 3,25 g (20,3 mmol) (R,S)-1,3-Dioxotetrahydrooxazol(3,4a]piperazin-7-carbonsäure-t-butylester portionsweise zugegeben. Nach 1 h bei 40 - 50 °C wurde mit Wasser und 0,1 N Salzsäure ausgeschüttelt. Nach dem Trocknen über Magnesiumsulfat wurde einrotiert und die zwei Diastereomeren mittels MPLC (EtOAc / MeOH = 5:1) getrennt und als farblose Öle isoliert.

### 3(R)-[1(S)-Phenylethylcarbamoyl]piperazin-1-carbonsäure-t-butylester

R_{f}= 0,74 (EtOAc / MeOH = 5:1)
¹H-NMR (CDCl₃, 400 MHz): δ 7,35 - 7,21 (m, 5 H),
7,20 - 7,12 (m, 1H),
5,15 - 5,06 ("quint", 1H),
4,08 - 4,01 ("dd", 1H),
3,85 - 3,65 (m, 1H),
3,31 - 3,25 ("dd", 1H),
3,06 - 2,82 (m, 3H),
2,76 - 2,67 ("t", 1H),
2,04 - 1,90 (m, 1H),
1,48 (d, J = 7,7 Hz, 3H),
1,44 (s, 9H).

¹³C-NMR(CDCl₃, 100 MHz): δ 169,92 (s),
154,42 (s),
142,96 (s),
128,45 (d),
127,08 (d),
125,83 (d),
79,80 (s),
58,02 (d),
48,13 (d),
46,2 (br, t),
43,86 (t),
43,4 (br, t),
28,15 (q),
21,74 (q).

### 3(S)-[1(S)-Phenylethylcarbamoyl]piperazin-1-carbonsäure-t-butylester

R_{f}= 0,56 (EtOAc / MeOH = 5:1)
¹H-NMR (CDCl₃, 400 MHz): δ 7,35 - 7,25 (m, 4H),
7,25 - 7,17 (m, 2H),
5,13 - 5,04 ("quint", 1H),
4,08 - 4,00 ("dd", 1H),
3,80 - 3,67 (m, 1H),
3,29 - 3,23 ("dd", 1H),
3,04 - 2,81 (m, 3H),
2,73 - 2,63 ("t", 1H),
2,04 - 1,90 (m, 1H),
1,46 (d, J = 7,7 Hz, 3H),
1,44 (s, 9H).

¹³C-NMR (CDCl₃, 100 MHz): δ 169,88(s),
154,33 (s),
142,92 (s),
128,31 (d),
126,97 (d),
125,80 (d),
79,68 (s),
57,90 (d),
48,09 (d),
47,0 (br, t),
43,72 (t),
43,5 (br, t),
28,06 (q),
21,63 (q).

### Beispiel 3

### Herstellung von 3(S)-[1(S)-Methoxycarbonyl-2-methylpropylcarbamoyl]piperazin-1-carbonsäure-t-butylester

Zu 0,50 g (1,95 mmol) (S)-1,3-Dioxotetrahydrooxazol[3,4a]piperazin-7-carbonsäüre-t-butylester und 0,67 g (4,00 mmol) L-Valinmethylester-hydrochlorid in 20 ml Dichlormethan wurde 0,44 g (4,35 mmol) Triethylamin unter Rühren zugetropft. Nach 15 min unter Rückfluß wurde auf Raumtemperatur abgekühlt, mit 1 N Salzsäure auf pH = 2 gestellt und mit 40 ml Essigsäureethylester extrahiert. Die Wasserphase wurde noch zweimal mit je 30 ml Essigsäureethylester extrahiert und die vereinigten organischen Phasen über Magnesiumsulfat getrocknet. Das Rohprodukt (0,63 g, 94 %) wurde zur vollständigen Charakterisierung chromatographisch gereinigt (Silicagel, EtOAc / MeOH = 5:1, R_{f} = 0,58) und 0,24 g der reinen Titelverbindung als farbloses Öl isoliert.
¹H-NMR (CDCl₃, 400 MHz): δ 7,38 -7,25 (m, 1 H),
4,54 - 4,50 ("dd", 1 H),
4,04 ("d", br, 1 H),
3,80 - 3,70 (m, 1 H),
3,73 (s, 3 H),
3,39 - 3,36 ("dd", 1 H),
3,11 - 3,05 ("dd", 1 H),
3,05 - 2,92 (m, 2 H),
2,85 - 2,75 (m, 1 H),
2,23 - 2,13 (m, 1 H),
2,00 - 1,92 (m, 1 H),
1,46 (s, 9 H),
0,95 (d, J = 6,9 Hz, 3 H),
0,92 (d, J = 6,8 Hz, 3 H).

¹³C-NMR (CDCl₃, 100 MHz): δ 172,29 (s),
171,27 (s),
154,67 (s),
80,13 (s),
58,44 (d),
56,81 (d),
52,11 (q),
46,60 (br, t),
43,96 (t),
43,90 (br, t),
31,11 (d),
28,41 (q),
19,12 (q),
17,86 (q).

### Beispiel 4

### Herstellung von 3-t-Butylcarbamoylpiperazin-1-carbonsäureallylester

Zu 3,00 g (10,0 mmol) Piperazin-1,2,4-tricarbonsäure-1,4-diallylester in 20 ml Methylenchlorid wurde 1,78 g (15,0 mmol) Thionylchlorid bei 22° C zugetropft. Anschliessend wurde 20 Stunden bei 22 °C weitergerührt. Zu dieser Reaktionslösung wurde innert 5 Minuten unter Kühlung 2,92 g (40 rnmol) t-Butylamin zugetropft. Die entstandenen Suspension wurde noch 3 Stunden bei 22 °C gehalten und dann mit 50 ml Wasser und 50 ml Methylenchlorid versetzt. Die Phasen wurden getrennt und die Wasserphase noch zweimal mit je 30 ml Methylenchlorid extrahiert. Trocknung der vereinigten organischen Phasen über Magnesiumsulfat ergab nach dem Eindampfen 2,30 g (85 %) der Titelverbindung als hellgelbes Öl. Zur vollständigen Charakterisierung wurde das Rohprodukt chromatographisch gereinigt.
¹H-NMR (DMSO-d₆, 400 MHz): δ 7,30 - 7,20 (m, 1 H),
5,98 - 5,85 (m, 1 H),
5,28 ("d", 1 H),
5,19 ("d", 1 H),
4,58 - 4,48 (m, 2 H),
3,90 - 3,82 (m, 1 H),
3,70 - 3,62 (m, 1 H),
3,10 - 3,05 ("dd", 1 H),
2,90 - 2,80 (m, 3 H),
2,59 - 2,50 (m, 2 H),
1,25 (s, 9 H).

## Patentansprüche

1. Verfahren zur Herstellung von 2-Piperazincarbonsäureamiden in Form seiner Enantiomeren oder seiner Enantiomerenmischungen der allgemeinen Formel 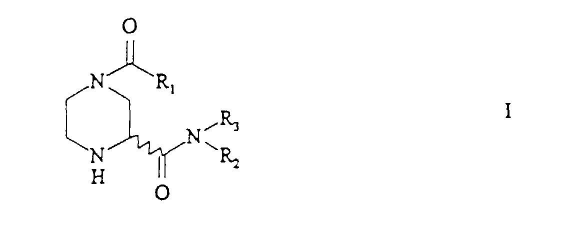 worin R₁ die Bedeutung hat von a) gegebenenfalls substituiertes Alkyl oder b) von -OR₄, worin R₄ gegebenenfalls substituiertes Alkyl, Alkenyl oder Aryl bedeutet oder c) von -NR₅R₆, worin R₅ Wasserstoff oder Alkyl und R₆ Alkyl bedeutet und
R₂ und R₃ gleich oder verschieden sind und die Bedeutung haben von Wasserstoff, gegebenenfalls substituiertes Alkyl, Alkenyl oder Aryl oder für den Rest einer Aminosäure oder eines Aminosäureesters stehen, dadurch gekennzeichnet, dass eine 2-Piperazincarbonsäure der Formel 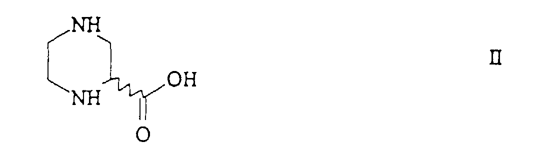 oder ein Salz davon zunächst in ein N-Acylderivat der allgemeinen Formel 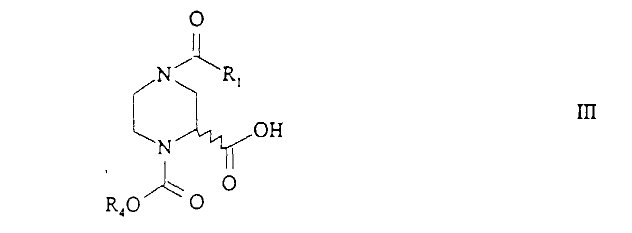 worin R₁ und R₄ die genannte Bedeutung haben, umgewandelt wird, dieses in einer zweiten Stufe in Gegenwart von einem Halogenierungsmittel in ein
Piperazincarbonsaureanhydrid der allgemeinen Formel 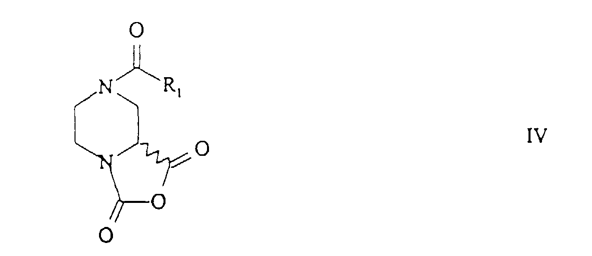 worin R₁ die genannte Bedeutung hat, cyclisiert wird und schliesslich mit einem Amin der allgemeinen Formel 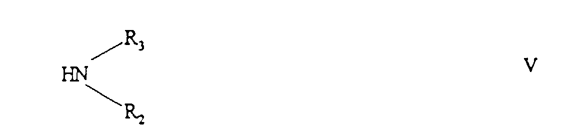 worin R₂ und R₃ die genannte Bedeutung haben, in das Endprodukt der allgemeinen Formel I umgesetzt wird.

2. Verfahren nach Patentanspruch 1, dadurch gekennzeichnet, dass R₁ die Bedeutung von -OR₄ hat.

3. Verfahren nach Patentanspruch 1 oder 2, dadurch gekennzeichnet, dass das N-Acylderivat der allgemeinen Formel III nicht isoliert wird.

4. Verfahren nach einem der Patentansprüche 1 bis 3, dadurch gekennzeichnet, dass als Halogenierungsmittel Thionylchlorid, Phosphorhalogenide, Phosgen oder Phosgenderivate eingesetzt werden.

5. Verfahren nach Patentanspruch 4, dadurch gekennzeichnet, dass Thionylchlorid verwendet wird.

6. Verfahren nach Patentanspruch 4 oder 5, dadurch gekennzeichnet, dass katalytische Mengen von 0,1 mol% bis 30 mol% eines N,N-disubstituierten Formamids bezogen auf das Halogenierungsmittel eingesetzt werden.

7. Verfahren nach einem der Patentansprüche 1 bis 6, dadurch gekennzeichnet, dass die Umsetzung in der zweiten Stufe bei 0° bis 100°C durchgeführt wird.

8. Verfahren nach einem der Patentansprüche 1 bis 7, dadurch gekennzeichnet, dass die Umsetzung mit dem Amin der allgemeinen Formel V in Gegenwart eines inerten Lösungsmittels oder in wässrigen alkalischen Lösungen bei einer Temperatur von -20°C bis Rückflusstemperatur des Lösungsmittels erfolgt.

9. Piperazincarbonsäureanhydride der allgemeinen Formel 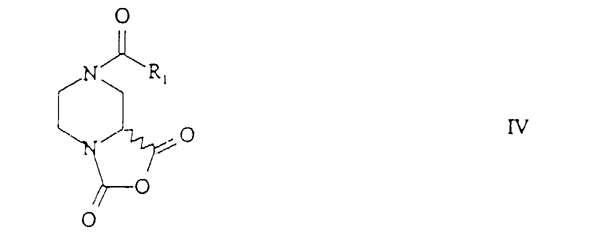 worin R₁ die Bedeutung hat von
a) gegebenenfalls mit Halogen oder Phenyl substituiertes (C₁-C₆-Alkyl,
b) von OR₄, worin R₄ gegebenenfalls mit Halogen oder Phenyl substituiertes (C₁-C₆)-Alkyl oder Alkenyl oder gegebenenfalls mit Halogen oder (C₁-C₆)-Alkyl substituiertes Phenyl oder Naphthyl bedeutet, oder
c) von -NR₅R₆, worin R₅ Wasserstoff oder (C₁-C₆)-Alkyl und R₆ (C₁-C₆)-Alkyl bedeutet.

10. (R)-1,3-Dioxotetrahydrooxazo[3,4-a]piperazin-7-carbonsäure-t-butylester.

11. (S)-1,3-Dioxotetrahydrooxazo[3,4-a]piperazin-7-carbonsäure-t-butylester.

12. (R)-1,3-Dioxotetrahydrooxazo[3,4-a]piperazin-7-carbonsäurebenzylester.

13. (S)-1,3-Dioxotetrahydrooxazo[3,4-a]piperazin-7-carbontäurebenpylester.

14. (R)-1,3-Dioxotetrahydrooxazo[3,4-a]piperazin-7-carbonsäureallylester.

15. (S)-1,3-Dioxotetrahydrooxazo[3,4-a]piperzzin-7-carbonsäureallylester.

## Claims

1. A process for the preparation of 2-piperazine carboxylic acid amides in the form of the enantiomers thereof or enantiomer mixtures thereof having the general formula 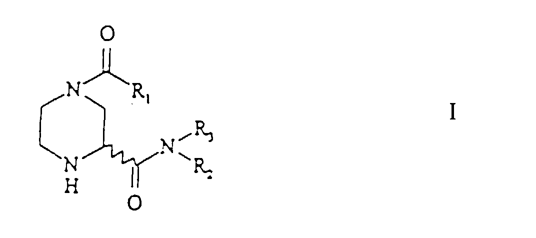 wherein R₁ has the meaning of a) optionally substituted alkyl or b) -OR₄, wherein R₄ means optionally substituted alkyl, alkenyl or aryl, or c) -NR₅R₆, wherein R₅ means hydrogen or alkyl and R₆ means alkyl and R₂ and R₃ are the same or different and have the meaning of hydrogen, optionally substituted alkyl, alkenyl or aryl or stand for the radical of an amino acid or of an amino acid ester, characterised in that a 2-piperazine carboxylic acid having the formula 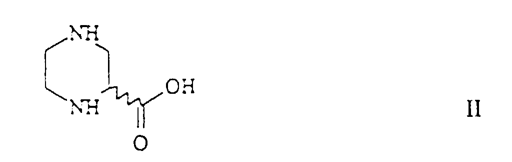 or a salt thereof is initially converted to an N-acyl derivative having the general formula 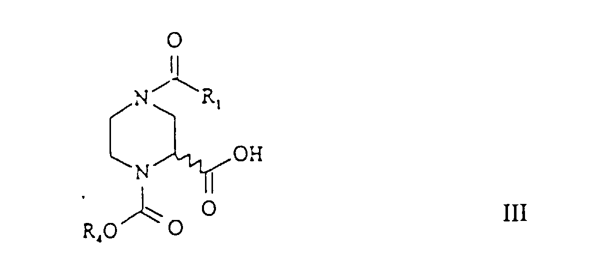 wherein R₁ and R₄ have the meaning given, said derivative is cyclised in a second step in the presence of a halogenating agent to a piperazine carboxylic acid anhydride having the general formula 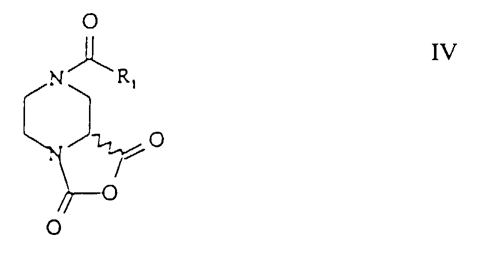 wherein R₁ has the meaning given, and is finally reacted with an amine having the general formula 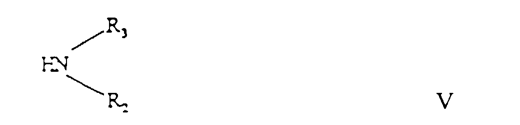 wherein R₂ and R₃ have the meaning given, to give the end product having the general formula I.

2. A process according to patent claim 1, characterised in that R₁ has the meaning of -OR₄.

3. A process according to patent claim 1 or 2, characterised in that the N-acyl derivative having the general formula III is not isolated.

4. A process according to one of patent claims 1 to 3, characterised in that halogenating agents used are thionyl chloride, phosphorus halides, phosgene or phosgene derivatives.

5. A process according to patent claim 4, characterised in that thionyl chloride is used.

6. A process according to patent claim 4 or 5, characterised in that catalytic amounts from 0.1 mole % to 30 mole % of an N,N-disubstituted formamide, based on the halogenating agent, are used.

7. A process according to one of patent claims 1 to 6, characterised in that the reaction in the second step is carried out at 0°C to 100°C.

8. A process according to one of patent claims 1 to 7, characterised in that the reaction with the amine having the general formula V is carried out in the presence of an inert solvent or in aqueous alkaline solutions at a temperature from -20°C to reflux temperature of the solvent.

9. Piperazine carboxylic acid anhydrides having the general formula 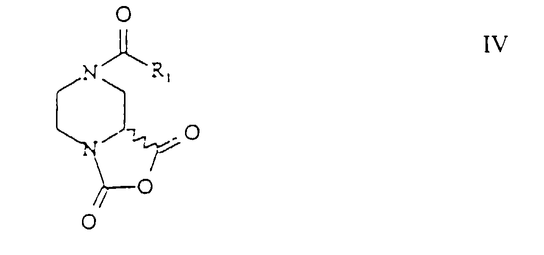 wherein R₁ has the meaning of
a) C₁-C₆-alkyl optionally substituted with halogen or phenyl,
b) OR₄, wherein R₄ means (C₁-C₆)-alkyl or alkenyl optionally substitutes with halogen or phenyl, or phenyl or naphthyl optionally substituted with halogen or (C₁-C₆)-alkyl, or
c) -NR₅R₆, wherein R₅ means hydrogen or (C₁-C₆)-alkyl and R₆ means (C₁-C₆)-alkyl.

10. (R)-1,3-dioxotetrahydrooxazo[3,4-a]piperazine-7-carboxylic acid-t-butyl ester.

11. (S)-1,3-dioxotetrahydrooxazo [3,4-a]piperazine-7-carboxylic acid-t-butyl ester.

12. (R)-1,3-dioxotetrahydrooxazo[3,4-a]piperazine-7-carboxylic acid benzyl ester.

13. (S)-1,3-dioxotetrahydrooxazo[3,4-a]piperazine-7-carboxylic acid benzyl ester.

14. (R)-1,3-dioxotetrahydrooxazo[3,4-a]piperazine-7-carboxylic acid allyl ester.

15. (S)-1,3-dioxotetrahydrooxazo[3,4-a]piperazine-7-carboxylic acid allyl ester.

## Revendications

1. Procédé de préparation de 2-pipérazinecarboxamides sous forme de leurs énantiomères ou de leurs mélanges d'énantiomères de formule générale 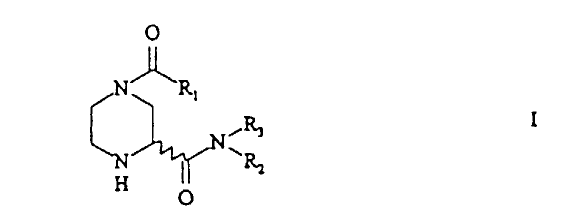 où R₁ a la signification a) d'alkyle éventuellement substitué ou b) de -OR₄, où R₄ représente alkyle, alcényle ou aryle éventuellement, substitué ou c) de -NR₅R₆ où R₅ représente l'hydrogène ou alkyle et R₆ représente alkyle et R₂ et R₃ sont identiques ou différents et on la signification de l'hydrogène, alkyle, alcényle ou aryle éventuellement substitué ou représentent le reste d'un acide aminé ou d'un ester d'acide aminé, caractérisé en ce qu'un acide 2-pipérazinecarboxylique de formule 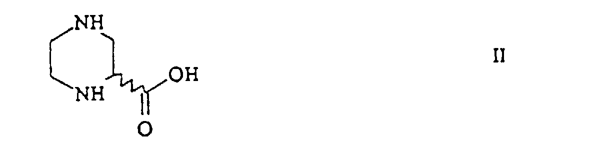 ou un sel de celui-ci est d'abord converti en un dérivé N-acylé de formule générale 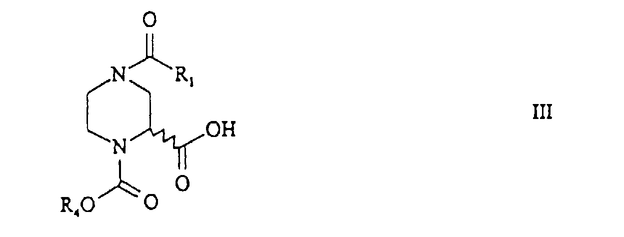 où R₁ et R₄ ont la signification citée, celui-ci est cyclisé dans une deuxième étape en présence d'un agent d'halogénation en un anhydride d'acide pipérazine-carboxylique de formule générale 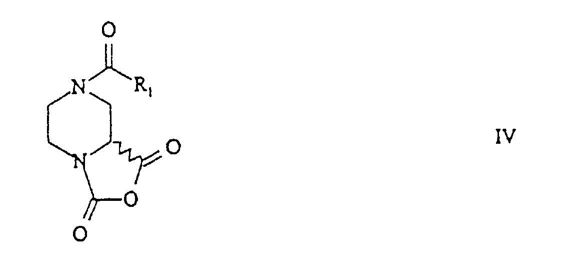 où R₁ a la signification citée, et enfin converti avec une amine de formule générale 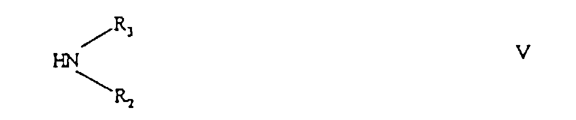 où R₂ et R₃ ont la signification citée, en le produit final de formule générale I.

2. Procédé selon la revendication 1 caractérisé en ce que R₁ a la signification de -OR₄.

3. Procédé selon la revendication 1 ou 2 caractérisé en ce que le dérivé N-acylé de formule générale III n'est pas isolé.

4. Procédé selon l'une des revendications 1 à 3 caractérisé en ce que l'on utilise comme agent d'halogénation le chlorure de thionyle, des halogénures de phosphore, le phosgène ou des dérivés du phosgène.

5. Procédé selon la revendication 4 caractérisé en ce que l'on utilise le chlorure de thionyle.

6. Procédé selon la revendication 4 ou 5 caractérisé en ce que l'on utilise des quantités catalytiques de 0,1 mol% à 30 mol% d'un formamide N,N-disubstitué par rapport à l'agent d'halogénation.

7. Procédé selon l'une des revendications 1 à 6 caractérisé en ce que la réaction dans la deuxième étape est conduite entre 0° et 100°C.

8. Procédé selon l'une des revendications 1 à 7 caractérisé en ce que la réaction avec l'amine de formule générale V a lieu en présence d'un solvant inerte ou dans des solutions aqueuses alcalines à une température de - 20°C à la température de reflux du solvant.

9. Anhydrides d'acides pipérazinecarboxyliques de formule générale 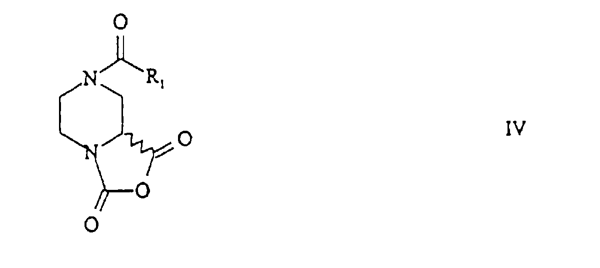 où R₁ a la signification
a) d'alkyle en C₁-C₆ éventuellement substitué par halogène ou phényle,
b) de OR₄ où R₄ signifie alkyle en C₁-C₆ ou alcényle éventuellement substitué par halogène ou phényle, ou phényle ou naphtyle éventuellement substitué par halogène ou alkyle en C₁-C₆, ou
c) de -NR₅R₆ où R₅ signifie l'hydrogène ou alkyle en C₁-C₆ et R₆ signifie alkyle en C₁-C₆.

10. (R)-1,3-dioxotétrahydrooxazo[3,4-a]pipérazine-7-carboxylate de t-butyle.

11. (S)-1,3-dioxotétrahydrooxazo[3,4-a]pipérazine-7-carboxylate de t-butyle.

12. (R)-1,3-dioxotétrahydrooxazo[3,4-a]pipérazine-7-carboxylate de benzyle.

13. (S)-1,3-dioxotétrahydrooxazo[3,4-a]pipérazine-7-carboxylate de benzyle.

14. (R)-1,3-dioxotétrahydrooxazo[3,4-a]pipérazine-7-carboxylate d'allyle.

15. (S)-1,3-dioxotétrahydrooxazo[3,4-a]pipérazine-7-carboxylate d'allyle.
